(19) Europäisches Patentamt
European Patent Office
Office européen des brevets

(11) **EP 2 868 279 A1**

(12) **EUROPEAN PATENT APPLICATION**

(43) Date of publication:
06.05.2015 Bulletin 2015/19

(51) Int Cl.:
**A61B 8/00** (2006.01)          *A61B 5/00* (2006.01)
**A61B 8/08** (2006.01)

(21) Application number: 14189130.9

(22) Date of filing: 16.10.2014

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 31.10.2013 US 201361898035 P

(71) Applicant: Canon Kabushiki Kaisha
Tokyo 146-8501 (JP)

(72) Inventors:
• Nagae, Kenichi
Tokyo, Tokyo 146-8501 (JP)

• Furukawa, Yukio
Tokyo, Tokyo 146-8501 (JP)
• Taku, Masakazu
Tokyo, Tokyo 146-8501 (JP)
• Nishihara, Hiroshi
Tokyo, Tokyo 146-8501 (JP)
• Kruger, Robert A.
Oriental, NC North Carolina 28571 (US)

(74) Representative: TBK
Bavariaring 4-6
80336 München (DE)

(54) **Subject information acquisition apparatus**

(57)     A subject information acquisition apparatus according to the present invention includes multiple first conversion elements (101) which receive photoacoustic waves. A direction in which reception sensitivity is highest differs between a first part of the first conversion elements (101), and a second part of the first conversion elements (101) which is different from the first part of the first conversion elements (101). Both are disposed on the supporting member (100) with the direction in which reception sensitivity is highest each facing toward a predetermined region. Also included is a plurality of second conversion elements (102) which are different from the first conversion elements (101) and which transmit acoustic waves to the subject (103) and each receive reflection waves reflected within the subject (103).

FIG. 1

**Description**

BACKGROUND OF THE INVENTION

Field of the Invention

The present invention relates to a subject information acquisition apparatus. More particularly, the present invention relates to a subject information acquisition apparatus which receives acoustic waves from a subject and acquires information regarding within the subject.

Description of the Related Art

Imaging of the inside of a subject, such as with photoacoustic imaging (PAI), is a current area of research. Photoacoustic imaging works by a subject being irradiated by pulsed light, and photoacoustic waves generated at tissue within the subject which has absorbed light energy are received (typically ultrasonic band acoustic waves). The received signals are then subjected to signal processing, and thus information of within the subject can be acquired.

Information acquired by photoacoustic waves differs depending on the wavelength of the light energy by which the subject is irradiated. For example, enhanced visualization of portions within the subject where blood is present can be obtained in a case where near-infrared light, which is well absorbed by hemoglobin, is used. If information indicating not only where blood is present but also indicating the structure of the tissue can be obtained when diagnosing using images obtained in this way, highly accurate diagnosis may be enabled due to the increased amount of information.

The ultrasound echo method used in ultrasound diagnosis apparatuses can be applied to acquisition to information indicating tissue structure. Accordingly, imparting a subject information acquisition apparatus with a function to receive photoacoustic waves and a function to transmit/receive acoustic waves such as ultrasound waves can be conceived useful in acquiring both functional information and structural information of within the subject.

International Publication No. 2010/030817 discloses an apparatus which uses a conversion element which receives photoacoustic waves, to also transmit/receive acoustic waves, as an example of such an apparatus.

In International Publication No. 2010/030817, one conversion element serves to perform both reception of photoacoustic waves and transmission/reception of acoustic waves. However, characteristics may differ between photoacoustic waves, and ultrasound waves transmitted/received in the ultrasound echo method (hereinafter also referred to simply as "acoustic waves"). Accordingly, there is demand for efficient reception of photoacoustic waves and transmission/reception of acoustic waves.

It has been found desirable to enable efficient reception of photoacoustic waves and transmission/reception of acoustic waves.

SUMMARY OF THE INVENTION

The present invention in its first aspect provides a subject information acquisition apparatus as specified in claims 1 to 20.

The present invention in its second aspect provides a subject information acquisition apparatus as specified in claim 21.

Further features of the present invention will become apparent from the following description of exemplary embodiments with reference to the attached drawings.

BRIEF DESCRIPTION OF THE DRAWINGS

Fig. 1 is a schematic diagram illustrating a subject information acquisition apparatus according to an embodiment.
Fig. 2 is a schematic plan view of a bowl portion.
Fig. 3 is a schematic diagram illustrating the trajectory of a supporting member.
Fig. 4 is a schematic diagram illustrating the structure of a second conversion element array.
Figs. 5A and 5B are schematic diagrams illustrating the structure of the second conversion element array.
Figs. 6A and 6B are schematic diagrams, Fig. 6A illustrating the trajectory of the supporting member, and Fig. 6B illustrating a variation example of the second conversion element array.
Figs. 7A and 7B are schematic diagrams, Fig. 7A illustrating acquisition timing of PA signals and US signals, and Fig. 7B illustrating the relationship between the placement of second conversion element arrays and acquisition timing.

Figs. 8A and 8B are schematic diagrams, Fig. 8A illustrating acquisition timing of PA signals and US signals, and Fig. 8B illustrating the relationship between the placement of second conversion element arrays and acquisition timing.

Figs. 9A and 9B are schematic diagrams illustrating the trajectory of the second conversion element array.

Figs. 10A through 10C are schematic diagrams illustrating the configuration of the second conversion element array.

Figs. 11A through 11C are schematic diagrams illustrating the positional relationship of a holding member and the second conversion element array.

DESCRIPTION OF THE EMBODIMENTS

Embodiments of the present invention will be described with reference to the drawings. Components which are the same will be denoted by the same reference numerals as a general rule, and description thereof will be omitted.

Note that in the present Specification, acoustic waves generated within the subject by optical absorption will be referred to as "photoacoustic waves" and acoustic waves transmitted from the conversion element will be referred to as "ultrasound waves", to facilitate description.

The subject information acquisition apparatus according to the present invention acquires characteristics information indicating characteristics values corresponding to each of multiple positions within the subject, using received signals of photoacoustic waves. The characteristics information acquired from the photoacoustic waves reflects the absorbance of light energy. Specifically, characteristics information acquired from photoacoustic waves includes characteristics information reflecting initial sound pressure of generated photoacoustic waves, light energy absorption density derived from the initial sound pressure, absorption factors, concentration of substance of which the tissue is composed, and so forth. Examples of concentration of substance include oxygen saturation, total hemoglobin concentration, concentration of oxyhemoglobin or deoxyhemoglobin, or the like. Characteristics information at multiple locations may be acquired as a two-dimensional or three-dimensional characteristics distribution. Characteristics distribution may be generated as image data.

The subject information acquisition apparatus also can receive ultrasound waves (reflection waves) which are the transmitted ultrasound waves reflected back from within the subject. Characteristics information acquired from received signals of reflected waves reflects difference in acoustic impedance within the subject. Specific examples of characteristics information difference acquired from reflected waves include morphologic information reflecting acoustic impedance within the subject, information indicating elasticity and viscosity of the tissue, and motion information such as bloodstream flow speed, that are derived from the acoustic impedance difference, and so forth. Characteristics information at multiple locations may be acquired as a two-dimensional or three-dimensional characteristics distribution. Characteristics distribution may be generated as image data.

Note that the subject information acquisition apparatus according to the following embodiments is primarily directed to diagnosis of malignancy, blood vessel disease, and so forth, in humans and animals, follow-up observation during chemotherapy, and so forth. Accordingly, anticipated diagnosis objectives are living subjects, specifically human and animal mammaries (e.g., a breast), and so forth.

First Embodiment

The following is a description of the configuration of the subject information acquisition apparatus and the processing flow thereof, according to a first embodiment.

Overall Apparatus Configuration

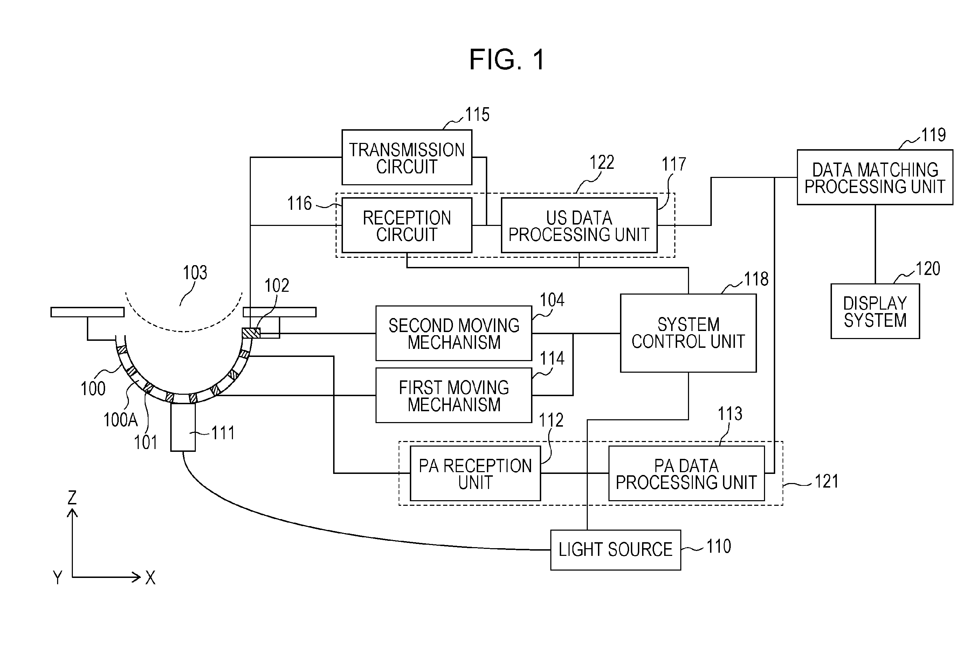

Fig. 1 is a schematic diagram illustrating the system configuration of the present embodiment. The subject information acquisition apparatus according to the present embodiment includes at least a light source 110, multiple first conversion elements 101 for receiving photoacoustic waves, a supporting member 100 for supporting multiple first conversion elements 101, a first moving mechanism 114 for moving the supporting member 100, and multiple second conversion elements 102.

A subject 103 is irradiated by light from the light source 110, via an optical transmission system 111. Photoacoustic waves are received by each of the multiple first conversion elements 101, and time-series reception signals (photoacoustic (PA) signals)) are output from the first conversion elements 101. The PA signals are input to a PA signal processing unit 121, with the PA signal processing unit 121 creating PA data based on the PA signals.

On the other hand, the second conversion elements 102 transmit ultrasound waves to the subject 103 following transmission signals from a transmission circuit 115. Reflection waves are received by the second conversion elements 102, and time-series reception signals (ultrasound (US) signals)) are output from the second conversion elements 102. The US signals are input to a US signal processing unit 122, with the US signal processing unit 122 creating US data.

The multiple second conversion elements 102 can be moved by the second moving mechanism 104. Note that in Fig. 1, the multiple second conversion elements 102 are arrayed in a direction perpendicular to the plane of the drawing.

A data matching processing unit 119 generates display data based on the PA data and US data, which is output to a display system 120.

Note that a feature of one form of the present invention is that the first conversion elements 101 and second conversion elements 102 are provided separately (not shared). This is because the characteristics of the photoacoustic waves which the first conversion elements 101 receive and the characteristics of the ultrasound waves which the second conversion elements 102 transmit/receive may be different. Differences in characteristics will be described in the detailed description of the components of the present embodiment.

Processing Flow Relating to Reception of Photoacoustic Waves

A processing flow regarding reception of photoacoustic waves will be described. First, the first moving mechanism 114 starts moving the supporting member 100 so as to move over a predetermined trajectory, following instructions from a system control unit 118.

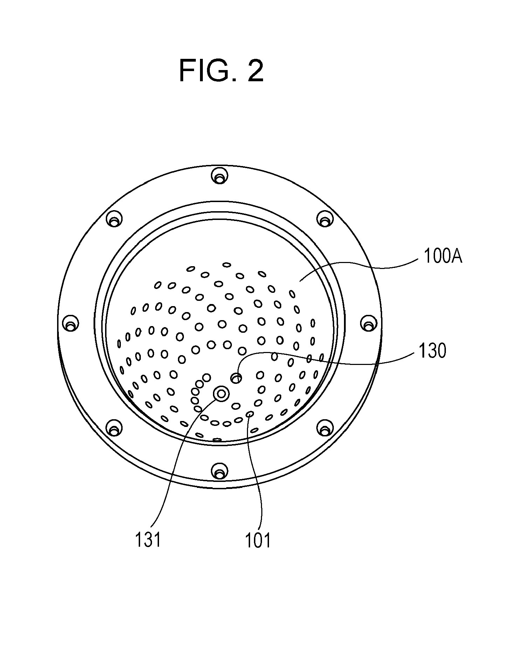

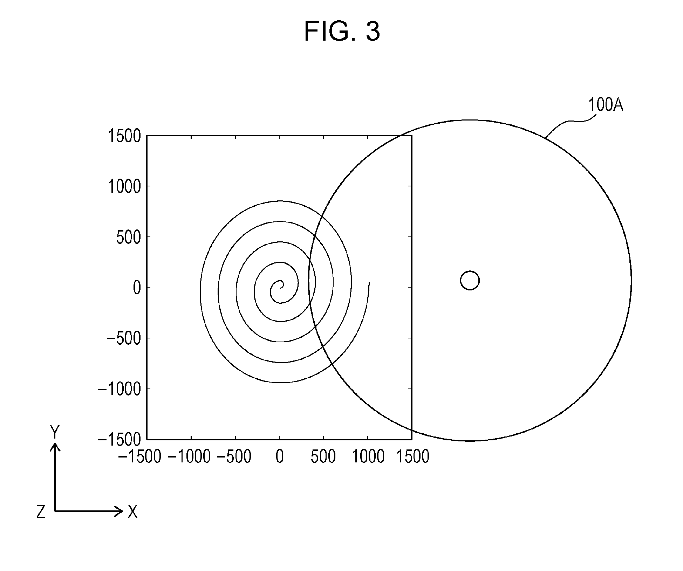

Note that here, the multiple first conversion elements 101 are provided in a three-dimensional spiral form on a bowl portion 100A of the supporting member 100, as illustrated in Fig. 2, and are configured to move as the supporting member 100 moves. Fig. 3 illustrates an example of the supporting member 100 moving over a two-dimensional spiral trajectory. Fig. 2 illustrates a plan view of the bowl portion 100A, and Fig. 3 illustrates the way in which the supporting member 100 moves so that the center of the bowl portion 100A traces a two-dimensional spiral when viewing the bowl portion 100A from above.

The light source 110 emits light at predetermined light-emission intervals according to instructions from the system control unit 118. Pulsed light emitted from the light source 110 at a certain timing while the supporting member 100 is moving passes through the optical transmission system 111 and the subject 103 is irradiated thereby. The light transmission speed is sufficiently fast, so the light emission time by the light source 110 can be handled as the same time as the time of irradiation of the subject by the light.

Part of the light energy which has propagated through the subject 103 is absorbed by light absorbents which absorb light of a predetermined wavelength (e.g., blood vessels which contain a great amount of hemoglobin), and thermal expansion of the light absorbents generates photoacoustic waves. The multiple first conversion elements 101 provided to the supporting member 100 receive these photoacoustic waves and convert into time-series reception signals (PA signals).

Note that the propagation speed of ultrasound waves (the speed of sound) is faster than the moving speed of the supporting member 100. Accordingly, the detection position where a certain first conversion element 101 (which will be referred to as first conversion element 101A) receives photoacoustic waves can be handled as being the same position at the position where the first conversion element 101A exists at the timing at which the subject 103 is irradiated by the pulsed light which has generated those photoacoustic waves. That is to say, the amount of movement of the supporting member 100 from the subject 103 being irradiated by the pulsed light up to the first conversion elements 101 receiving the photoacoustic waves can be considered to be negligible, so the timing of irradiating the pulsed light and the timing of receiving the photoacoustic waves can be handled as being the same. The supporting member 100 has multiple first conversion elements 101, so the positions of each of the multiple first conversion elements 101 can be identified by identifying the position of the supporting member 100.

The light source 110 emits light at a predetermined cycle, and the supporting member 100 moves at a predetermined speed, so at a light irradiation time different from the earlier light irradiation time, the first conversion elements 101 receive photoacoustic waves at a detection position different from the detection position at the earlier light irradiation time.

The PA signals output from the multiple first conversion elements 101 at each reception timing are sequentially input to the PA signal processing unit 121. A PA reception circuit 112 performs amplification and AD conversion of the PA signals, and transmits digitized PA signals to a PA data processing unit 113. The PA data processing unit 113 performs image reconstruction to acquire characteristics distribution of an optional region (target region), based on the signals from the PA reception circuit 112. Characteristics distribution of the target region can be acquired as a set of voxel data in a case of acquiring three-dimensional information, or as a set of pixel data in a case of acquiring two-dimensional information. The acquired characteristics distribution is transmitted to the data matching processing unit 119 as PA data.

The above-described flow allows PA data of the target region to be obtained efficiently and accurately. Note that the PA data processing unit 113 does not have to use all PA signals stored in memory in the PA data processing unit 113 in a case of acquiring PA data in an optional target region.

Processing Flow Relating to Transmission/Reception of Ultrasound Waves

Next, a processing flow relating to transmission/reception of ultrasound waves will be described. An example where multiple second conversion elements 102 are provided to the supporting member 100 will be described here. Specifically, the multiple second conversion elements 102 are arrayed on an outer periphery side region from the upper end portion of the bowl portion 100A (that is to say, the end portion at the opening side) as illustrated in Fig. 1. Multiple second conversion elements 102 are arrayed in a direction perpendicular to the plane of the drawing in Fig. 1. The configuration is such that the multiple second conversion elements 102 move as the supporting member 100 moves.

First, the first moving mechanism 114 starts moving the supporting member 100 so as to move over a predetermined trajectory, following instructions from the system control unit 118. An example where movement is over a two-dimensional spiral trajectory will be described here, in the same way as with the two-dimensional spiral trajectory in the case of receiving photoacoustic waves. The transmission circuit 115 transmits transmission signals according to the instructions of the system control unit 118 to the second conversion elements 102. The second conversion elements 102 transmit ultrasound pulses to the subject 103 based on the input transmission signals. The ultrasound waves scattered and reflected within the subject 103 reach the second conversion elements 102 again as reflection waves. The second conversion elements 102 receive the reflection waves and convert into time-series reception signals (US signals).

Note that the propagation speed of ultrasound waves is faster than the moving speed of the supporting member 100, so the detection position where a certain second conversion element 102 (which will be referred to as second conversion element 102A, for example) receives reflection waves can be handled as being the same position at the position where the second conversion element 102A exists at the timing at which the ultrasound waves are transmitted. That is to say, the amount of movement of the supporting member 100 from the ultrasound waves being transmitted to the second conversion elements 102 receiving the reflection waves can be considered to be negligible.

The multiple second conversion elements 102 perform transmission/reception of ultrasound waves necessary to acquire a tomographic image of a predetermined range at each detection position as the supporting member 100 moves. US signals output from the multiple second conversion elements 102 at each detection position are sequentially input to the US signal processing unit 122. A US reception circuit 116 performs amplification and AD conversion of the US signals, and transmits digitized US signals to a US data processing unit 117. The US data processing unit 117 performs reception beam forming processing based on the signals from the US reception circuit 116, and thus can acquire characteristics distribution corresponding to the multiple tomographic images. The acquired characteristics distributions are transmitted to the data matching processing unit 119 as US data. The data matching processing unit 119 outputs display data to the display system 120, based on the PA data and US data that have been input.

In the above description, photoacoustic waves are received while moving the supporting member 100 over a predetermined trajectory, and thereafter the supporting member 100 is moved over the same trajectory while performing transmission/reception of ultrasound waves at multiple detection positions. That is to say, moving for reception of photoacoustic waves and moving for transmission/reception of ultrasound waves are performed separately. However, reception of photoacoustic waves and transmission/reception of ultrasound waves may be performed during one same movement of the supporting member 100. That is to say, the supporting member 100 may be moved while alternately performing reception of photoacoustic waves and transmission/reception of ultrasound waves at each detection position.

Next, the components of the subject information acquisition apparatus according to the present embodiment will be described in detail.

Light Source 110

The light source 110 preferably is a pulsed light source capable of emitting nanosecond to microsecond order pulsed light. Specifically, a pulse width of around 1 to 100 nanoseconds is used to effectively generate photoacoustic waves. The wavelength is preferably around 600 nm to 1100 nm. Pulsed lasers such as Nd:YAG lasers and alexandrite lasers are specific light source examples of preferably used lasers. Ti:sapphire lasers or optical parametric oscillators (0P0) using Nd:YAG laser as excitation light, and so forth, may also be used. Other than these, semiconductor lasers and the light may also be used. Light is transmitted from the light source 110 to the subject 103 by the optical transmission system 111. The optical transmission system 111 may use optical elements such as lenses, mirrors, optical fiber, and so forth.

Supporting Member 100

The supporting member 100 is a member which fixes and supports multiple first conversion elements 101. The supporting member 100 has a bowl portion 100A having a spherical face, as illustrated in Figs. 1 and 2.

When inspecting a subject 103, the subject 103 is inserted into the space within the supporting member 100 from the opening of the subject supporting member 100. An acoustic transmission medium such as water is injected into the bowl portion 100A of the supporting member 100 from an inlet 130 situated at the bottom portion of the bowl portion 100A, so as to fill between the bowl portion 100A and the subject 103. Also, a thin cup-shaped holding member (omitted from illustration) which holds the subject 103 may be provided as described in International Publication No. 2010/030817. In this case, the acoustic transmission medium is filled in between both the bowl portion 100A and the holding member, and the holding member and the subject 103. Further, a light emitting unit 131 is provided to the bottom portion of the bowl portion 100A, so that the subject 103 is irradiated by light from the light emitting unit 131 that has been transmitted through the optical transmission system 111.

First Conversion Elements 101

Any sort of conversion elements may be used for the first conversion elements 101 as long as being capable of receiving acoustic waves and converting into electric signals, including conversion elements such as piezoelectric elements employing the piezoelectric phenomenon, conversion elements employing optical resonance, electrostatic capacitance conversion elements such as capacitive micromachined ultrasonic transducers (CMUT) and the like, and so forth.

The multiple first conversion elements 101 are arrayed on the supporting member 100, such that the multiple first conversion elements 101 are arrayed in a three-dimensional spiral with the reception faces thereof following the spherical face of the bowl portion 100A, as illustrated in Fig. 2. Arraying so as to following the spherical face enables a configuration where the direction of each first conversion element 101 where reception sensitivity is high faces a predetermined region.

Typically, the normal line direction of the reception face (surface) of a conversion element is the direction where the reception sensitivity is the highest. Accordingly, an array following a spherical face such as illustrated in Fig. 2 enables the direction of each of the first conversion elements 101 where the reception sensitivity is higher than a predetermined level to be directed toward around the center of the curvature of the hemisphere-shaped bowl portion 100A (predetermined region). The multiple first conversion elements 101 are particularly preferably arrayed such that the directions where the sensitivity is the highest intersect around the center of curvature of the bowl portion 100A.

The present embodiment enables higher resolution at a region where the direction of each of the first conversion elements 101 where the reception sensitivity is higher than a predetermined level is directed. In the present specification, such a region where reception can be performed at high sensitivity will be referred to as a "high-sensitivity region"; high-sensitivity regions are consequently high-resolution regions. Note that a preferable range for a high-resolution region is a range from a highest-resolution point to a point where the resolution is half of the highest resolution. Specifically, diameter r in the following Expression (1) indicates the diameter of a high-resolution region,

$$r = \frac{r_0}{\phi_d} \cdot \sqrt{(R^2 - R_H^2)} \qquad \cdots (1)$$

where R is permissible resolution, $R_H$ is highest resolution, $r_0$ is the diameter of a sphere where the conversion elements are to be placed, and $\Phi_d$ is the diameter of each first conversion element 101.

The array of the multiple first conversion elements 101 in the present embodiment is not restricted to the example such as illustrated in Fig. 2; an array where a predetermined high-sensitivity region can be formed is sufficient. An array where a predetermined high-sensitivity region can be formed is an array in which acoustic waves can be received with higher sensitivity as compared to a case where the directions of each of the first conversion elements 101 where the reception sensitivity is highest are parallel. The high-sensitivity region determined by the array of the multiple first conversion elements 101 is formed at a region where the subject 103 is assumed to be situated at the time of performing an inspection.

Specifically, of the multiple first conversion elements 101, at least two first conversion elements 101 are arrayed so that the directions of the highest reception sensitivity thereof preferably face the predetermined region.

That is to say, of the multiple first conversion elements 101, preferably a part of the first conversion elements 101, and first conversion elements 101 different from the part of the first conversion elements 101, have different directions where the respective reception sensitivities are highest, and the directions where the respective reception sensitivities are highest face the predetermined region. More preferably, the first conversion elements 101 are arrayed such that the directions where the respective reception sensitivities are highest in at least a part of the first conversion elements 101 intersect.

Thus, by the directions where the respective reception sensitivities are highest facing the predetermined region, the photoacoustic waves generated at the predetermined region can be received with higher sensitivity as compared to a case where the directions of each of the first conversion elements 101 where the reception sensitivity is highest are parallel. Consequently, the resolution of images at the predetermined region can be improved over a case where the directions of each of the first conversion elements 101 where the reception sensitivity is highest are parallel.

Such an array can also be said to be an array where, of the multiple first conversion elements 101, the directivity axes (axes following the directions of highest sensitivity) of at least part of the first conversion elements 101 converge.

Such an array can also be said to be an array where the reception faces of the first conversion elements 101 face the inward side of the supporting member 100. That is to say, in a case where the first conversion elements 101 are provided to a supporting member having a curved surface such as a spherical face or the like, the reception faces of the first conversion elements 101 are arrayed following the surface thereof toward the center of curvature. Moreover, in a case where the first conversion elements 101 are provided to a supporting member having a combination of multiple planes (the angles formed by the planes are preferably obtuse angles) as the face thereof, the reception faces are arrayed following the inner surface (surface on the recessed side).

Note that in the present Specification, the term "spherical face" includes spherical faces other than a face on a true sphere, and accordingly includes spherical faces having an opening, such as a hemisphere-shape and so forth. Also included are faces having unevenness on a surface of a level which can be deemed to be a "spherical face", planes on ellipsoids (a shape formed by expanding an ellipse three-dimensionally; the surface is a quadratic surface), and so forth.

First Moving Mechanism 114

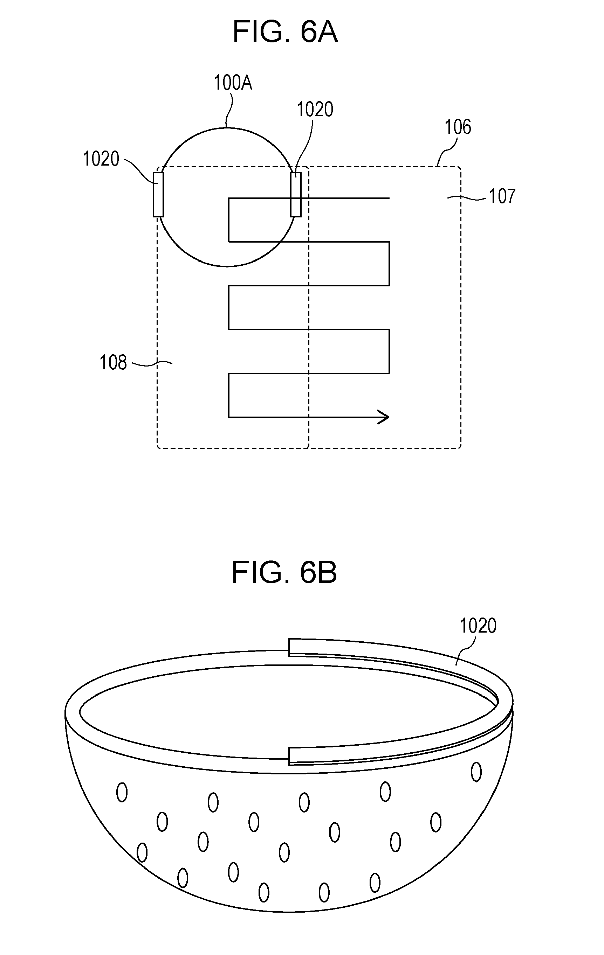

The first moving mechanism 114 can change the relative position of each of the multiple first conversion elements 101 as to the subject 103, by moving the supporting member 100. Specifically, the first moving mechanism 114 can move the supporting member 100 along a two-dimensional spiral trajectory such as illustrated in Fig. 3 or a linear trajectory (see later-described Fig. 6A).

Moving along a two-dimensional spiral trajectory or a linear trajectory enables the relative position to be changed between the high-sensitivity region determined by the array of the multiple first conversion elements 101 and the subject 103. Accordingly, variation of resolution within a single image can be reduced. That is to say, the high-sensitivity region exists at different positions for each reception timing of photoacoustic waves due to the motion of the supporting member 100, and as a result, the high-sensitivity region can be enlarged overall. The movement of the supporting member 100 may be a continuous movement over the trajectory, or may be a step-and-repeat type movement where the supporting member 100 is temporarily stopped at each point (each detection position) on the trajectory before going to the next point.

Movement is not limited to two-dimensional movement (on an XY plane) and may include three-dimensional movement (in an XYZ space). Specifically, the supporting member 100 may be moved vertically such that the high-sensitivity region is moved in the depth direction (Z direction) of the subject 103.

Further, the first moving mechanism 114 may rotationally move (rotate) the supporting member 100 centrally on a predetermined axis of the supporting member 100 (e.g., center axis of the spherical face). Even when performing rotational movement, resolution is improved in a case of a three-dimensional spiral array of the first conversion elements 101 such as illustrated in Fig. 2.

The first moving mechanism 114 can be configured using a motor such as a stepping motor or the like, a motorized stage having guides or the like, and so forth. In a case of providing a second moving mechanism 104, the second moving mechanism 104 can also be configured using the same members as the first moving mechanism 114.

PA Signal Processing Unit 121

The PA signal processing unit 121 processes reception signals output from the first conversion elements 101, thereby generating a distribution of characteristics information reflecting the absorbance of light energy as PA data. The PA signal processing unit 121 according to the present embodiment includes a PA reception circuit 112 and a PA data processing unit 113.

The PA reception circuit 112 includes an amplifier for amplifying reception signals from the first conversion elements 101, and an A/D converter for digitizing analog reception signals.

The PA data processing unit 113 reconstructs images using signals output from the PA reception circuit 112. Known reconstruction techniques, such as universal back projection (UBP) described in US Patent No. 5,713,356, filtered back projection (FBP), and so forth, may be used as image reconstruction techniques. Performing image reconstruction enables a distribution to be generated on two-dimensional or three-dimensional coordinates axes (distribution corresponding to the space within the subject). The PA data processing unit 113 includes memory, a workstation running a central processing unit (CPU) or graphics processing unit (GPU), a field programmable gate array (FPGA) chip, or the like. Memory is typically configured including computer readable storage mediums such as read-only memory (ROM), random access memory (RAM), a hard disk, and so forth. A storage unit is not restricted to being configured including one storage medium, and may be configured including multiple storage mediums. Transmission Circuit 115

The transmission circuit 115 is a signal generating circuit which generates transmission signals (pulse signals) of a waveform having a delay time (phase) and amplitude corresponding to the transmission direction of ultrasound waves, following control signals from the system control unit 118. The transmission signals are input to the second conversion elements 102, with ultrasound waves being transmitted from the second conversion elements 102 as pulse waves. The transmission circuit 115 includes a multiplexer, switch, and so forth. Second Conversion Elements 102

In the same way as with the first conversion elements 101, any sort of conversion elements may be used for the second conversion elements 102 as long as being capable of receiving acoustic waves and converting into electric signals, including conversion elements such as piezoelectric elements employing the piezoelectric phenomenon, conversion elements employing optical resonance, electrostatic capacitance conversion elements such as CMUTs and the like, and so forth.

Also in the present embodiment, not all of the second conversion elements 102 have to be used when performing one ultrasound wave transmission. That is to say, a part of the second conversion elements 102 (a second conversion element group) may be used to perform one ultrasound wave transmission (i.e., transmission beam forming). In this case, an electronic scan is preferably performed, where transmission of ultrasound wave beams is repeated while sequentially switching the second conversion elements 102 used in the transmission beam forming.

In the present embodiment, the first conversion elements 101 and the second conversion elements 102 are not shared, but rather provided according to separate usages. In particular, the second conversion elements 102 according to the present embodiment are disposed at different positions from the first conversion elements 101. Fig. 1 illustrates the multiple second conversion elements 102 being provided at a region on the outer periphery side from the opening of the bowl portion 100A of the supporting member 100. However, the multiple second conversion elements 102 may be provided at the end portion at the opening. The term "end portion" as used in the present Specification is not restricted to the edge of the opening and includes regions nearby the edge. Specifically, this includes regions on the inner periphery side, as long as not impeding photoacoustic wave paths when the first conversion elements 101 receive photoacoustic waves.

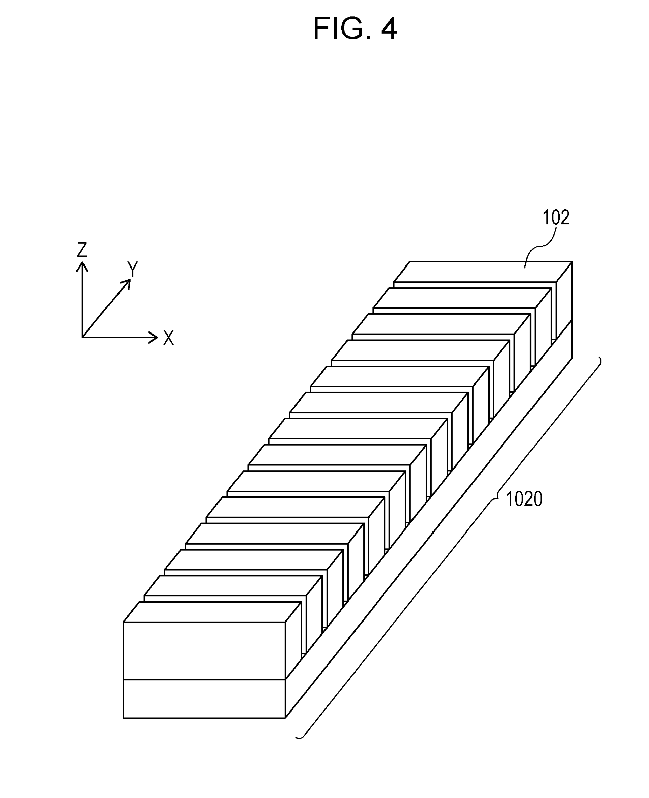

The array of the multiple second conversion elements 102 is in particular a 1D array, arrayed one-dimensionally on a plane as illustrated in Fig. 4. In the following description, the configuration where multiple second conversion elements 102 are arrayed will be called a "second conversion element array 1020".

Note that the second conversion element array 1020 according to the present embodiment may be arranged such that the multiple second conversion elements 102 are arrayed as an arc on a plane, as described later with reference to Fig. 6B. The array is not restricted to a 1D array, and may be configurations called 1.5D array, 1.75D array, or 2D array.

The reception face of each second conversion element 102 faces upwards (Z direction), and transmits ultrasound waves toward the subject positioned above. Note however, an arrangement may be made where the direction in which the reception faces of the second conversion elements 102 faces is inclined a predetermined angle (e.g., 10 degrees or more but 25 degrees or less) from the Z direction, so as to face toward the center side of the spherical face of the bowl portion 100A. Thus, efficient ultrasound wave transmission can be performed by inclining the direction where the sensitivity of the second conversion elements 102 is the highest (or the ultrasound wave beam transmission direction) a predetermined angle (e.g., 10 degrees or more but 25 degrees or less) from the Z direction.

Further, while the direction in which the multiple second conversion elements 102 are arrayed (longitudinal direction of the second conversion element array 1020) has been described as the direction perpendicular to the plane of the drawing in Fig. 1 (i.e., the tangent line direction of a circle formed by the opening portion of the bowl portion 100A), the direction of the longitudinal direction is not restricted to this. For example, an arrangement may be made where the multiple second conversion elements 102 are arrayed in the radial direction of the circle formed by the opening portion of the bowl portion 100A. Particularly, in a case of moving the second conversion element array 1020 by moving the supporting member 100 over a two-dimensional spiral trajectory, the longitudinal direction of the second conversion element array 1020 and the tangent line direction of the circle formed by the opening portion preferably intersect. Thus, imaging can be effectively performed near the center of the trajectory.

The second conversion elements 102 are illustrated disposed above the first conversion elements 101 (toward the subject side) in Fig. 1. The reason why this position is preferable is that the second conversion elements 102 are conversion elements which transmit ultrasound waves and receive the reflected waves. Specifically, the time which it takes for the second conversion elements 102 to transmit ultrasound waves and receive the returning reflected waves is longer than the time it takes for the first conversion elements 101 to receive the photoacoustic waves from the point of light irradiation, with regard to a region at the same depth. That is to say, in order to receive a subject region at the same depth at a reception time which does not greatly differ from the time of the photoacoustic waves, the second conversion element array 1020 is preferably situated at a position closer to the subject than the multiple first conversion elements 101. Being closer to the subject also enables the frame rate of acquiring tomographic images to be raised as well.

Further, providing the second conversion elements 102 at the end portion of the opening side of the bowl portion 100A, or at a region on the outer side from the opening portion is preferable, since propagation of the photoacoustic waves which the first conversion elements 101 receive is not impeded.

Also, in a case of performing transmission beam forming, the ultrasound waves are transmitted such that the ultrasound wave beams converge at a predetermined location of the subject, and are reflected at a range on the ultrasound wave beam. In a case of performing transmission beam forming with the array of the multiple second conversion elements 102 being the same array as the that of the multiple first conversion elements 101 arrayed on the spherical face, the acquired ultrasound image may be different from an ultrasound image acquired by a general linear electronic scan. Accordingly, the multiple second conversion elements 102 are preferably each arrayed within a two-dimensional plane, to avoid giving the physician or the like, who is the user, a sense of incongruity with regard to the ultrasound image.

There are many cases where the transmitted/received ultrasound waves and the photoacoustic waves have different frequency characteristics. Specifically, there are cases where the center frequency of the photoacoustic waves is lower than the center frequency of the ultrasound waves. Accordingly, conversion elements with a higher center frequency than the first conversion elements 101 are preferably used as the second conversion elements 102. Accordingly, conversion elements of which the center frequency is higher than the first conversion elements 101 are preferably used for the second conversion elements 102. Using conversion elements having a high center frequency enables transmission/reception of ultrasound waves to be performed efficiently. Specifically, conversion elements having a center frequency in the range of 7 to 15 MHz is preferable for the second conversion elements 102, and conversion elements having a center frequency in the range of 1 to 10 MHz is preferable for the first conversion elements 101.

Since it is sufficient for the present invention for the first conversion elements 101 and the second conversion elements 102 to be provided separately, the second conversion elements 102 may be disposed in a three-dimensional spiral, separately from the first conversion elements 101, which is described in a latter embodiment. The second conversion elements 102 according to the present embodiment may also serve as sensor to confirm whether or not the subject is situated at a predetermined position by transmission/reception of ultrasound waves.

US Signal Processing Unit 122

The US signal processing unit 122 generates the distribution of characteristics information reflecting difference in audio impedance as US data, by processing the reception signals output from the second conversion elements 102. The US signal processing unit 122 according to the present embodiment includes the US reception circuit 116 and US data processing unit 117.

The US reception circuit 116 includes a switch, an amplifier for amplifying reception signals from the second conversion elements 102, and an A/D converter for digitizing analog reception signals. Note that the number of channels of the US reception circuit 116 may not necessarily be the same as the number of second conversion elements 102. Typically, the number of channels of the US reception circuit 116 is smaller than the number of second conversion elements 102. For example, in a case where beam forming is performed in increments of 32 second conversion elements 102 out of 256 second conversion elements 102, 32 will suffice as the number of channels of the US reception circuit 116. The US reception circuit 116 and the PA reception circuit 112 may partially be shared.

The US data processing unit 117 is a processing unit which performs reception beam forming processing, and includes a phasing block, an envelope detection processing block, and a processing block which performs various types of filter processing.

Phasing (delay & sum) means processing to subject signals output from the US reception circuit 116 to phase adjustment based on information relating to the delay time of reflection waves from the predetermined position to each second conversion elements 102, and subsequently perform addition thereof. Note that phase adjustment includes reading reception signals of addresses corresponding to delay time, from memory storing the reception signals for each channel in time-series.

Signals after phasing are input to the envelope detection block as scanning wire signals. Scanning wire signals indicate signals on ultrasound wave beams that have been transmitted, and signals indicating the intensity of reflection waves from multiple positions existing on a single scanning wire are arrayed thereupon in time-series. A B-mode image displayed on a general ultrasound diagnosis apparatus is equivalent to a characteristics distribution image where as many envelopes of these scanning wire signals are arrayed as there are the multiple scanning wires.

Adaptive beam forming processing may be performed as the acquisition method for the characteristics distribution, besides the above-described reception beam forming processing. In adaptive beam forming, parameters such as phase, weighting, and so forth, are adaptively changed in accordance to the reception signals, reception signals of desired waves arriving from a target direction or position are selectively extracted, and reception signals of other unnecessary waves are suppressed.

Particularly, the Capon method, which is a type of adaptive beam forming processing is a method which performs processing on multiple input signals so as to minimize the output intensity (electric power intensity) in a state where sensitivity as to a direction of interest or a position of interest is fixed. This is also called directionally constrained minimization of power (DCMP) or minimum variance method.

This type of adaptive beam forming processing is effective in improving spatial resolution. "M. SASSO et al., Medical Ultrasound Imaging Using The Fully Adaptive Beamformer, Proc. Acoustics, Speech Signal Process volume 2, pp. 489-492 (Mar. 2005)" describes a technique to raise the resolution in a direction perpendicular to the depth direction (scanning wire direction).

Further, the frequency domain interferometry (FDI) method and Capon method may be combined. "Frequency Domain Interferometry (FDI) method. Hirofumi Taki, Kousuke Taki, Takuya Sakamoto, Makoto Yamakawa, Tsuyoshi Shiina and Toru Sato: Conf Proc IEEE Eng Med Biol Soc. 2010;1: 5298-5301" describes a technique to improve resolution in the depth direction, by applying the FDI method to the Capon method.

Further, the back projection method, which is the same as the image reconstruction of PA data at the PA data processing unit 113, may be applied as a US data (US characteristics distribution) acquisition method.

The US data processing unit 117 includes memory, a workstation running a CPU or GPU, an FPGA chip, or the like. Memory is typically configured including computer readable storage mediums such as ROM, RAM, a hard disk, and so forth. A storage unit is not restricted to being configured including one storage medium, and may be configured including multiple storage mediums. The US data processing unit 117 and the PA data processing unit 113 may also be partially shared.

Further, the system control unit 118 and data matching processing unit 119 also can be configured of memory, a CPU, GPU, and so forth.

The data matching processing unit 119 generates display data for display on the display system 120, using PA data and US data. Specifically, the data matching processing unit 119 can combine coordinate axes of photoacoustic characteristics distribution and ultrasound wave characteristics distribution, create color maps for each of photoacoustic characteristics distribution and ultrasound wave characteristics distribution, and so forth. The data matching processing unit 119 can also generate display data of images where the photoacoustic characteristics distribution and ultrasound wave characteristics distribution are superimposed or displayed in tandem. Of course, the photoacoustic characteristics distribution and ultrasound wave characteristics distribution may be displayed separately.

The display system 120 is a display apparatus where images are displayed based on the display data output from the data matching processing unit 119. The display system 120 can be configured using a liquid crystal display (LCD), cathode ray tube (CRT), organic electroluminescence (EL) display, or the like. Note that the display system 120 may be of a configuration being provided separately from the subject information acquisition apparatus and connected thereto, rather than being included in the subject information acquisition apparatus.

Variation Example of Second Conversion Element Array

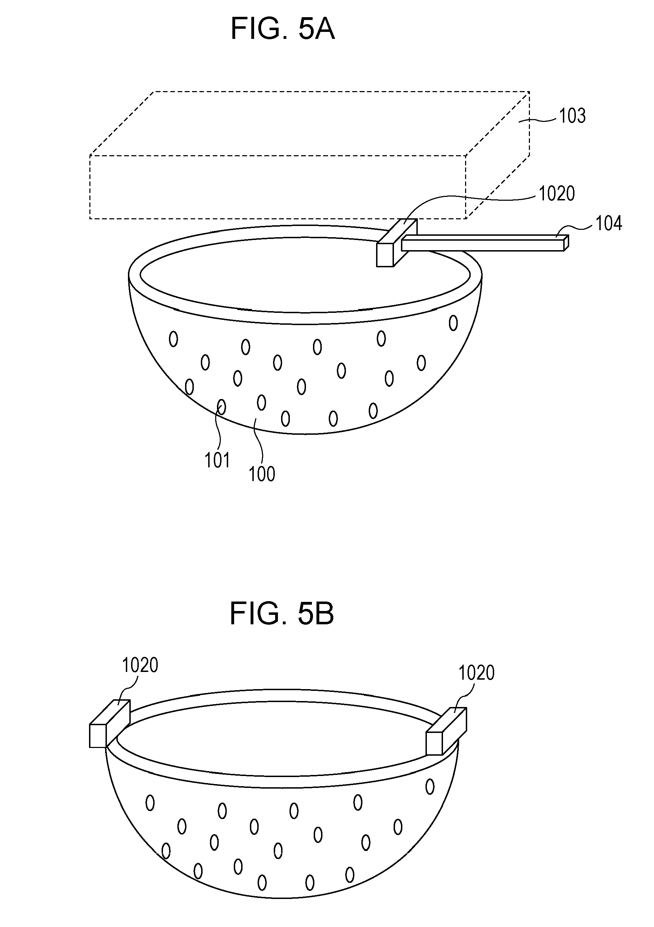

Next, a variation example of the second conversion element array 1020 will be described. Figs. 5A and 5B are schematic diagrams illustrating placement variation examples of the second conversion element array 1020.

The second conversion element array 1020 illustrated in Fig. 5A can move independently from the movement of the supporting member 100 by the second moving mechanism 104. That is to say, the second moving mechanism 104 can move the second conversion element array 1020 over a different trajectory at a different speed from the movement of the first conversion elements 101. For example, the second moving mechanism 104 can move the second conversion element array 1020 in a vertical direction as to the array direction of the second conversion elements 102, thereby maximizing the imaging region of the second conversion element array 1020 (corresponding to the acquisition region of ultrasound images).

Thus, due to the second conversion elements 102 being movable independent from the movement of the first conversion elements 101, ultrasound images can be acquired efficiently. The movement speed can also be changed according to the image quality level necessary for the ultrasound images.

Next, an example where multiple second conversion element arrays 1020 are provided will be described with reference to Fig. 5B. Fig. 5B is a schematic diagram, illustrating a configuration where the multiple second conversion elements 102 have been divided into two groups (two second conversion element arrays 1020). A second conversion element array 1020 serving as a first group, and a second conversion element array 1020 serving as a second group, are disposed at positions different from each other, at an outer periphery side region from the opening portion of the bowl portion 100A (symmetrically as to the center axis of the spherical face of the bowl portion 100A).

As illustrated in Fig. 5B, in a case where the multiple second conversion elements 102 are divided into multiple groups, wide-range ultrasound images can be acquired with a small amount of movement. Particularly, in a case of a configuration where the second conversion element array 1020 is moved by moving the supporting member 100, the supporting member 100 itself has to be moved over a wide range in order to acquire ultrasound images up to the end of the subject if only one second conversion element array 1020 has been provided. On the other hand, providing multiple second conversion element arrays 1020 on the outer periphery side region of the bowl portion 100A, the movement range of the supporting member 100 can be made smaller, increase in the size of the apparatus can be suppressed, and inspection time can be reduced. Further, the time for acquiring ultrasound images can be reduced as compared to one second conversion element array 1020 performing transmission/reception of ultrasound waves at each position.

The number of the second conversion element arrays 1020 is not restricted to two, and an arrangement may be made where four second conversion element arrays 1020 are provided at the outer periphery side region from the opening portion of the bowl portion 100A, with a positional relationship so as to have a spatial phase difference of 90 degrees centered on the axis. Alternatively, two second conversion element arrays 1020 may be provided with a positional relationship so as to have a spatial phase difference of 90 degrees centered on the axis.

Next, an example of the trajectory over which the second conversion element array 1020 moves will be described with reference to Fig. 6A. Fig. 6A is a schematic diagram illustrating the movement trajectory of the supporting member 100. A supporting member 100 provided with two second conversion element arrays 1020 as in Fig. 5B moves following the arrow illustrated in Fig. 6A. The second conversion element arrays 1020 perform transmission/reception of ultrasound waves at multiple detection positions along the trajectory.

As the supporting member 100 moves, the multiple first conversion elements 101 fixed to the bowl portion 100A also move. Irradiation by light and reception of photoacoustic waves by the first conversion elements 101 are performed at multiple detection positions along the trajectory.

Now, the range over which photoacoustic images can be acquired is determined by the light quantity distribution within the subject, and the reception directionality and array of the first conversion elements 101. For example, by setting the diameter of light irradiation of the subject surface to be around 6 cm, and the high-sensitivity region by the first conversion elements 101 to be a region having a diameter of around 6 cm near the center of the spherical face, a photoacoustic image of a spherical region around 6 cm in diameter can be acquired for each light irradiation. The high-sensitivity region in Fig. 6A moves along with the movement of the supporting member 100.

On the other hand, the second conversion element array 1020 which transmits/receives the ultrasound waves is switched between the two second conversion element arrays 1020 according to the detection position on the trajectory, by instruction from the system control unit 118. This operation carries out transmission/reception of ultrasound waves at multiple detection positions within an observation region 107 to the right side by the right-hand second conversion element array 1020, and carries out transmission/reception of ultrasound waves at multiple detection positions within an observation region 108 to the left side by the left-hand second conversion element array 1020. Ultrasound images of the observation region 107 and observation region 108 are then generated based on the reception signals from these second conversion element arrays 1020.

Thus, ultrasound images can be acquired for a region equal to or greater than the photoacoustic image acquisition range, by using two second conversion element arrays 1020. Moreover, there may be periods where transmission/reception switching of ultrasound waves is not performed among the two second conversion element arrays 1020. That is to say, both of the two second conversion element arrays 1020 may perform transmission/reception of ultrasound waves nearby the boundary between the observation region 107 and observation region 108. Ultrasound images nearby the boundary may be created using reception signals from the two second conversion element arrays 1020. For example, image data acquired by each of the second conversion element arrays 1020 may be synthesized to configure ultrasound images near the boundary, or reception signals acquired by each of the second conversion element arrays 1020 may be synthesized to configure ultrasound images near the boundary.

Next, another example of the second conversion element array 1020 will be described with reference to Fig. 6B. Fig. 6B has the second conversion element array 1020 formed as an arc, disposed on the rim of the bowl portion 100A. This array also allows the observation region by the multiple second conversion elements 102 to overlap the observation region by the multiple first conversion elements 101.

Second Embodiment

Next, a second embodiment will be described. An example where acquisition of PA signals and US signals is performed alternately will be described. The same components as those described in the first embodiment can be used for the subject information acquisition apparatus according to the present embodiment. Accordingly, description of portions which are the same as the first embodiment will be omitted.

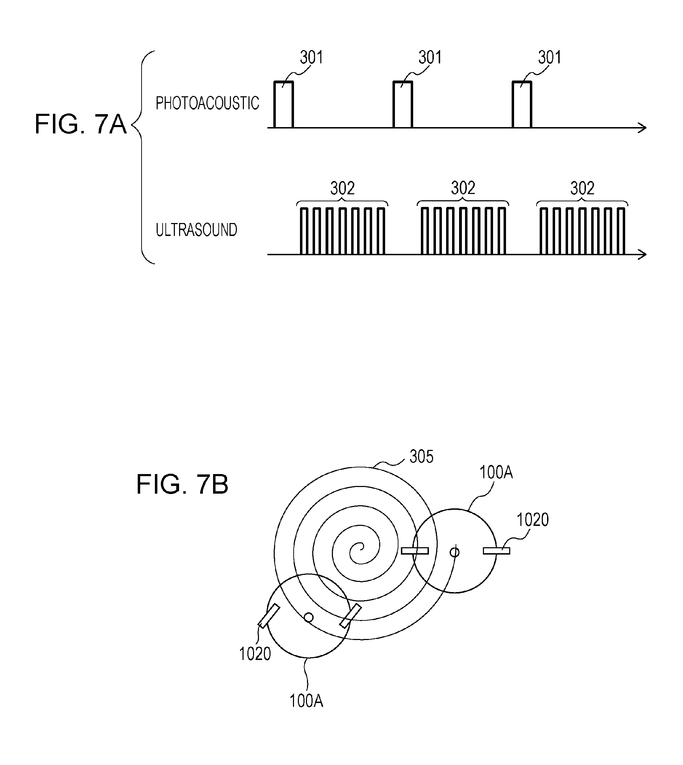

Fig. 7A is a schematic diagram illustrating acquisition timing of PA signals and US signals in the present embodiment, where a period 301 represents a PA signal acquisition period, and a period 302 represents a US signal acquisition period. Fig. 7A illustrates the subject 103 being intermittently irradiated by the pulsed light at a predetermined cycle (typically 10 Hz or 20 Hz), and PA signals are output from the first conversion elements 101 for each pulse of light.

On the other hand, in the periods 302 between the PA signal acquisition periods 301, transmission of ultrasound waves and reception of reflected waves is performed by the second conversion elements 102, and US signals are output from the second conversion elements 102. Note that transmission of the ultrasound waves may be performed multiple times in a period 302, as illustrated in Fig. 7A. Here, one set of reception of photoacoustic waves corresponding to period 301 and transmission/reception of ultrasound waves corresponding to period 302 is performed at each of the multiple detection positions on the moving trajectory.

The transmission/reception timing of ultrasound waves and the reception timing of photoacoustic waves can be kept from overlapping by the system control unit 118 performing such timing control, whereby deterioration of images due to mutual interference between the signals can be suppressed. Data acquisition efficiency can be improved since transmission/reception of ultrasound waves is performed in between reception of photoacoustic waves, so the overall inspection time can be kept from becoming long.

Next, an example of the array of second conversion element arrays 1020 at the US signal acquisition timing will be described. Fig. 7B is a schematic diagram illustrating the relation between the array of second conversion element arrays 1020 and the moving trajectory. In this example, the second conversion element arrays 1020 move relative to the supporting member 100 during the moving period of the supporting member 100, in accordance with the position of the bowl portion 100A on a trajectory 305. Specifically, the angles of the second conversion element arrays 1020 as to the supporting member 100 change during the movement of the supporting member 100, such that the longitudinal direction of the second conversion element arrays 1020 intersect (preferably orthogonally) the movement direction of the supporting member 100.

In a case of using 1D linear arrays for the second conversion element arrays 1020, a tomographic image acquired by transmission/reception of ultrasound waves by the second conversion element array 1020 at a certain detection position is a tomographic image parallel to the longitudinal direction of the 1D linear array. Consideration will now be given to a case where the angle of the second conversion element arrays 1020 change on the supporting member 100 such that the longitudinal direction is perpendicular to the movement direction of the supporting member 100, and a case otherwise.

First, a case will be considered where the angles of the second conversion element arrays 1020 change such that the longitudinal direction is perpendicular to the movement direction (e.g., rotating on the supporting member 100). In this case, the three-dimensional region of ultrasound images which can be acquired per unit time increases, due to the second conversion element arrays 1020 moving to multiple detection positions. On the other hand, in a case where the supporting member 100 moves but the positions of the second conversion element arrays 1020 on the supporting member 100 do not move, the range of three-dimensional region of ultrasound images which can be acquired per unit time is narrower than the former ultrasound images. For example, if the longitudinal directions of the second conversion element arrays 1020 are 45 degrees off as to the movement direction of the supporting member 100, the three-dimensional region of the ultrasound images acquired in the end is $1/\sqrt{2}$.

Accordingly, the second conversion element arrays 1020 preferably move with the installation angles as to the supporting member 100 changing, so that the longitudinal directions intersect the direction of movement. Movement of the second conversion element array 1020 may be performed by a newly provided third moving mechanism, or the second moving mechanism 104 performing this task as well.

Further, the present embodiment is not restricted to a method of changing the installation angle of the second conversion element arrays 1020 on the supporting member 100 as in the example described above, and other methods may be used to cause the moving direction and the longitudinal direction to intersect. For example, a configuration may be made where the longitudinal direction of the second conversion element arrays 1020 and the movement direction intersect by the supporting member 100 itself rotating while moving. In the case of this example, the second conversion element arrays 1020 do not need to move with respect to the supporting member 100.

As described above, a configuration where the longitudinal direction of the second conversion element arrays 1020 intersect (preferably orthogonally) the movement direction of the supporting member 100 enables the acquisition range of ultrasound images per unit time to be increased, and efficiency improves. While an example of a two-dimensional spiral has been illustrated in Fig. 7B for the trajectory 305, the above-described advantage can be obtained by the longitudinal direction of the second conversion element arrays 1020 and the moving direction of the supporting member 100 intersecting, regardless of the trajectory.

Note however, that the positional relationship where the longitudinal directions of the second conversion element arrays 1020 intersect the moving direction is a preferable example, and the present embodiment is not necessarily restricted to the supporting member 100 or second conversion element arrays 1020 moving to achieve an intersecting positional relationship.

Also, in a case where the supporting member 100 linearly moves such as the raster-scan-like trajectory in Fig. 6A, the angle between the main scan of the raster scan (movement in the direction where the movement distance is longer) and the longitudinal direction of the second conversion element array 1020 does not change. This sort of linear movement is preferably, since the second conversion element arrays 1020 do not have to move relative to the supporting member 100.

Variation Example of PA Signal and US Signal Acquisition Timings

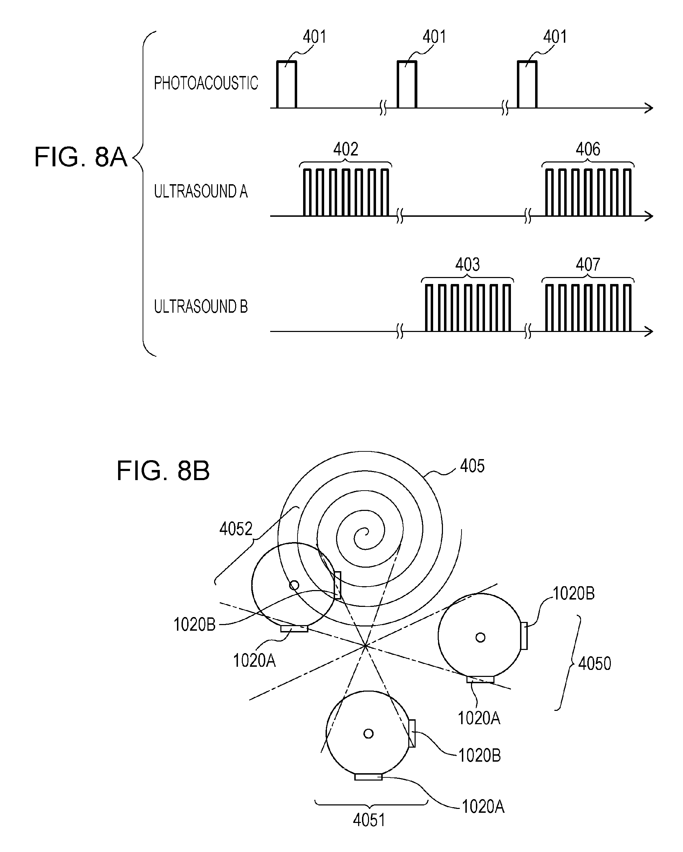

Next, a variation example of PA signal and US signal acquisition timings will be described. Fig. 8A is a schematic diagram illustrating PA signal and US signal acquisition timings according to the present embodiment, and Fig. 8B is a schematic diagram illustrating the relationship between the placement of a second conversion element array 1020A and a second conversion element array 1020B, and a movement trajectory 405 of the supporting member 100. The two second conversion element arrays 1020 are provided at a 90 degrees spatial phase difference in Fig. 8B.

A period 401 represents a PA signal acquisition period in which the subject 103 is intermittently irradiated by the pulsed light at a predetermined cycle (typically 10 Hz or 20 Hz), and PA signals are output from the first conversion elements 101 for each pulse of light. The supporting member 100 moves over the trajectory 405, and the multiple first conversion elements 101 receive photoacoustic waves at each of the multiple detection positions on the trajectory 405.

On the other hand, period 402 and period 406 represent periods in Fig. 8B in which transmission of ultrasound waves and reception of reflected waves is performed by the second conversion element array 1020A, and US signals are output from the second conversion element array 1020A. Period 403 and period 407 represent periods in which transmission of ultrasound waves and reception of reflected waves is performed by the second conversion element array 1020B, and US signals are output from the second conversion element array 1020B. That is to say, the system control unit 118 controls driving relating to transmission/reception by the second conversion element array 1020A and second conversion element array 1020B according to the position on the trajectory 405.

Fig. 8B illustrates space divided into 45 degrees each, with the center of the spiral trajectory 405 as a reference. For example, transmission/reception of ultrasound waves is performed by the second conversion element array 1020A where the relationship between moving direction and longitudinal direction is close to perpendicular while the supporting member 100 is moving over the trajectory through an angle range 2050, and the second conversion element array 1020B is not driven. Period 402 indicates acquisition of US signals during this period.

Transmission/reception of ultrasound waves is performed by the second conversion element array 1020B where the relationship between moving direction and longitudinal direction is close to perpendicular while the supporting member 100 is moving over the trajectory through an angle range 2051, and the second conversion element array 1020A is not driven. Period 403 indicates acquisition of US signals during this period.

Also, transmission/reception of ultrasound waves is performed by both the second conversion element array 1020A and second conversion element array 1020B while the supporting member 100 is moving over the trajectory through an angle range 2052, since angle between moving direction and longitudinal direction is about the same. Period 406 and period 407 indicate acquisition of US signals during this period.

As described above, multiple second conversion element arrays 1020 are provided in this example, and the second conversion element array 1020 to perform transmission/reception of ultrasound waves can be selected according to the position on the trajectory. That is to say, the second conversion elements 102 are selectable in increments of groups. This sort of control improves efficiency, since transmission/reception of ultrasound waves is performed in a state where the moving direction and the longitudinal direction of the second conversion element arrays 1020 are close to perpendicular.

Third Embodiment

Next, a third embodiment will be described. An example will be described in the present embodiment where a mode in which movement is performed while acquiring PA signals by the first conversion elements 101 at multiple detection positions on the trajectory, and a mode in which movement is performed while acquiring US signals at multiple detection positions on the trajectory. The same components as those described in the first embodiment can be used for the subject information acquisition apparatus according to the present embodiment. Accordingly, description of portions which are the same as the first embodiment will be omitted.

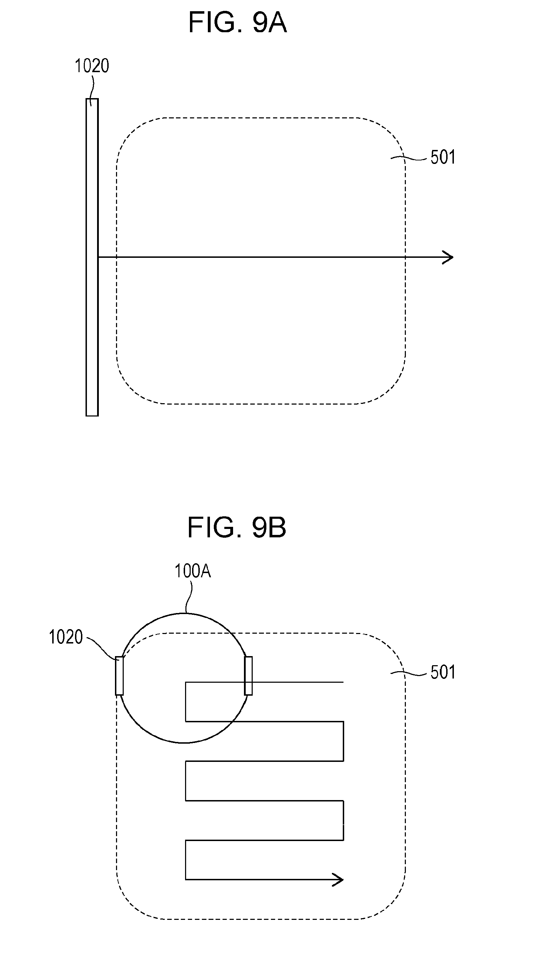

Fig. 9A is a schematic diagram illustrating movement of a second conversion element array 1020 according to the present embodiment. In this example, the second conversion element array 1020 is moved over the trajectory in the direction of the arrow while acquiring US signals at multiple detection positions, after the PA signal acquiring mode for generating photoacoustic images within an observation region 501 has been completed. Note that the length in the longitudinal direction of the second conversion element array 1020 in Fig. 9A is longer than the diameter of the opening portion of the bowl portion 100A. The second conversion element array 1020 is preferably longer than the diameter of the region where the subject is to be situated (e.g., a cup serving as a holding member to hold the subject). A second conversion element array 1020 configured thus can acquire ultrasound images of the observation region 501 (e.g., corresponding to the entire region of the subject) just by being moved one time in the direction of the arrow.

Fig. 9B is a schematic diagram illustrating another example of the present embodiment. In this example, the mode for acquiring US signals at multiple detection positions on the trajectory while moving the supporting member 100 in the direction of the arrow in Fig. 9B, after the PA signal acquiring mode for generating photoacoustic images within an observation region 501 has been completed. In the case of Fig. 9B, the longitudinal direction of the second conversion element array 1020 and the main scanning direction of the trajectory intersect, so ultrasound images can be efficiently acquired.

As described above, according to the present embodiment, the system control unit 118 can execute a mode for moving while acquiring PA signals at multiple detection positions on the trajectory, and a mode for acquiring US signals, at different periods. In this case, movement can be performed suitable for imaging characteristics of each of reception of photoacoustic waves and reception of ultrasound waves, which is advantageous.

Note that by moving the second conversion element array 1020 along with the supporting member 100 in the US signal acquisition mode enables the moving mechanism to be shared, thereby reducing the size of the apparatus. Note however, that the second conversion element array 1020 may be moved by a second moving mechanism independent from the supporting member 100. Further, the US signal acquisition mode may be performed first, and the PA signal acquisition mode performed thereafter.

Fourth Embodiment

Next, a fourth embodiment will be described. An example will be described in the present embodiment regarding an example where the second conversion element array 1020 is provided to the bowl portion 100A, or in a space on the inner side of the bowl portion 100A. Description of portions which are of the same configuration as those of the subject information acquisition apparatus described in the first through third embodiments will be omitted in the present embodiment as well.

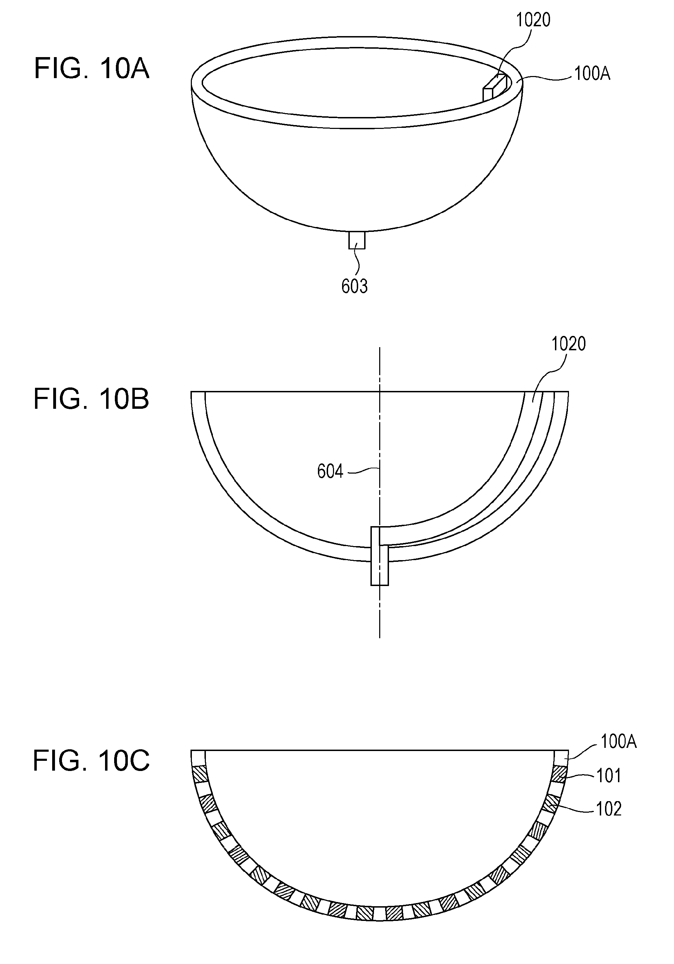

Figs. 10A and 10B are schematic diagrams illustrating the configuration of the second conversion element array 1020 according to the present embodiment. An arc-shaped second conversion element array 1020 is disposed on the inner side of the bowl portion 100A where multiple first conversion elements 101 are disposed. The second conversion element array 1020 is rotationally moved around a center axis 604 of the sphere in Fig. 10B, by a rotational shaft 603. In the case of such an embodiment, the region where the obtained photoacoustic images and ultrasound images overlap is greater, so efficient imaging is enabled.

Fig. 10C is a schematic diagram illustrating a separate example of the present embodiment. In this example, multiple second conversion elements are disposed on the bowl portion 100A of the supporting member 100 where multiple first conversion elements 101 are disposed. In the case of such an embodiment, the region where the obtained photoacoustic images and ultrasound images overlap is greater, so efficient imaging is enabled.

Note that the center frequency of the first conversion elements 101 and the center frequency of the second conversion elements 102 are made to differ in the present embodiment as well.

Fifth Embodiment

Next, a fifth embodiment will be described. An example will be described in the present embodiment regarding the relationship between the second conversion element array 1020 and a holding member holding the subject. Description of portions which are of the same configuration as those of the subject information acquisition apparatus described in the first through third embodiments will be omitted in the present embodiment as well.

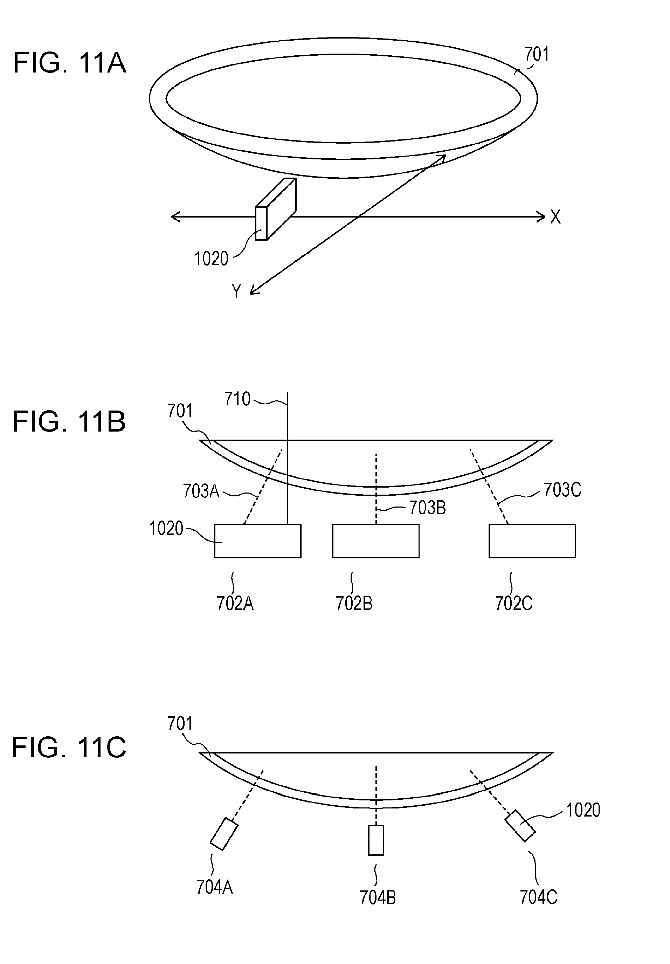

Fig. 11A is a schematic diagram illustrating the positional relationship in the present embodiment between a cup-shaped holding member 701 which holds the subject, and the second conversion element array 1020. The subject is positioned on the inner side of the holding member 701 (upper side in Fig. 11A) during the inspection. The second conversion element array 1020 moves over a predetermined trajectory on an XY plane, and performs transmission/reception of ultrasound waves at multiple detection positions on the trajectory.

Fig. 11B is a schematic illustration of a Y-direction cross-section of Fig. 11A. In a case where at least part of the holding member 701 has a curvature, the relative angle between the second conversion element array 1020 and the holding member 701 changes as the second conversion element array 1020 moves. For example, in a case where the second conversion element array 1020 is at position 702A, transmission/reception of ultrasound waves is performed in a perpendicular direction (710) as to the reception face of the second conversion element array 1020 (represented by the upper edge of the second conversion element array 1020 in Fig. 11B). In this case, the transmitted ultrasound beam is input to the holding member 701 at an angle which is not perpendicular to the holding member 701.

Now description will be made regarding a case where the ultrasound beam is input to the holding member 701 at an angle which is not perpendicular. The holding member 701 preferably has a certain level of mechanical strength, so there is the possibility that a material which is harder than the subject such as a living subject (that is to say, a material with fast acoustic wave propagation speed) may be used. Accordingly, there is the possibility of total reflection of the ultrasound waves in a case where the incident direction of the ultrasound waves and the direction of a perpendicular line at the surface of the holding member 701 is a certain angle or greater, e.g., an incident angle of 40 degrees or more. In a case where there is total reflection of the ultrasound waves, the ultrasound waves do not reach the subject, and the insider of the subject cannot be observed.

Accordingly, in the present embodiment, input to the holding member 701 is preferably performed such that an angle between the perpendicular line at the surface of the holding member 701 and the incidence direction of the ultrasound waves is smaller than the total reflection angle, which is to say, an angle closer to perpendicular than the total reflection angle as to the holding member 701. Also, in a case where ultrasound waves are input to the holding member 701 at an angle other than perpendicular, there is a possibility that refraction of the ultrasound waves may occur due to change in the acoustic velocity. From the aspect of suppression of distortion of the image due to this refraction, the ultrasound waves are preferably input to the holding member 701 at an even smaller incidence angle, which is to say an angle closer to being perpendicular to the holding member 701.

Accordingly, in the present embodiment, in a case where the second conversion element array 1020 is at the position 702A, the ultrasound beam is transmitted in the direction 703A, which is at an angle direction closer to perpendicular to the holding member 701. Also, in a case where the second conversion element array 1020 is at the position 702B, the ultrasound beam is transmitted in the direction 703B. Further, in a case where the second conversion element array 1020 is at the position 702C, the ultrasound beam is transmitted in the direction 703C. Performing such transmission control enables efficient ultrasound wave transmission/reception to be performed.

Fig. 11C is a schematic illustration of an X-direction cross-section of Fig. 11A. In a case where at least part of the holding member 701 has a curvature, the relative angle between the second conversion element array 1020 and the holding member 701 changes, in the same way as in the Y-direction cross-section.

In a case where the second conversion element array 1020 is at the position 704A in Fig. 11C, ultrasound waves are transmitted to the holding member 701 at an angle closer to perpendicular, by changing the attitude (angle as to the supporting member 100) of the second conversion element array 1020. In the same way, in a case where the second conversion element array 1020 is at the position 704B or position 704C, ultrasound waves can be transmitted to the holding member 701 at an angle closer to perpendicular relative to the surface of the holding member 701, by changing the attitude of the second conversion element array 1020. This attitude changing is performed by the second moving mechanism which has received instructions from the system control unit 118.

Accordingly, ultrasound waves can be transmitted to the holding member 701 at an angle greater than the total reflection angle, thus enabling efficient transmission/reception of ultrasound waves.

The present embodiment has thus been described regarding a technique where the transmission direction of the ultrasound waves is controlled without changing the attitude of the second conversion element array 1020, as illustrated in Fig. 11B, and a technique of controlling the attitude of the second conversion element array 1020 as illustrated in Fig. 11C. However, the incident angle of the ultrasound waves may be controlled using only the former, or the incident angle of the ultrasound waves may be controlled using only the latter. Also, the transmission direction of the ultrasound waves may be controlled using a 2D array second conversion element array 1020 arrayed in a two-dimensional matrix, in which case a mechanism for performing attitude control of an ultrasound probe can be omitted, whereby costs of the apparatus can be suppressed.

According to an embodiment of the present invention, reception of photoacoustic waves and transmission/reception of acoustic waves can be efficiently performed.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

A subject information acquisition apparatus according to the present invention includes multiple first conversion elements (101) which receive photoacoustic waves. A direction in which reception sensitivity is highest differs between a first part of the first conversion elements (101), and a second part of the first conversion elements (101) which is different from the first part of the first conversion elements (101). Both are disposed on the supporting member (100) with the direction in which reception sensitivity is highest each facing toward a predetermined region. Also included is a plurality of second conversion elements (102) which are different from the first conversion elements (101) and which transmit acoustic waves to the subject (103) and each receive reflection waves reflected within the subject (103).

## Claims

1. A subject information acquisition apparatus comprising:

   a light source (110) configured to generate light;
   a plurality of first conversion elements (101), each of which receives photoacoustic waves generated within a subject (103) by the subject (103) being irradiated by the light;
   a supporting member (100) configured to support the plurality of first conversion elements (101); and
   a plurality of second conversion elements (102) which are different from the first conversion elements (101) and which transmit acoustic waves to the subject (103) and each receive reflection waves reflected within the subject (103);
   wherein a direction in which reception sensitivity is highest differs between, out of the plurality of first conversion elements (101), a first part of the first conversion elements (101) and a second part of the first conversion elements (101) which is different from the first part of the first conversion elements (101), both being disposed on the supporting member (100) with the direction in which reception sensitivity is highest each facing toward a predetermined region.

2. The subject information acquisition apparatus according to Claim 1, further comprising:

   a first moving mechanism (114) configured to move the supporting member (100);
   wherein the first moving mechanism (114) moves the supporting member (100), to change a relative position between the plurality of first conversion elements (101) and the subject (103).

3. The subject information acquisition apparatus according to Claim 1,
   wherein the supporting member (100) includes a bowl portion (100A) which has a spherical face;
   and wherein the plurality of first conversion elements (101) are arrayed on the supporting member (100) in a three-dimensional spiral form, with the reception faces of the first conversion elements (101) following the spherical face.

4. The subject information acquisition apparatus according to Claim 2,
   wherein the first moving mechanism (114) moves the supporting member (100) along a trajectory which has a two-dimensional spiral form.

5. The subject information acquisition apparatus according to Claim 2,
   wherein the first moving mechanism (114) rotationally moves the supporting member (100) centrally on a predetermined axis.

6. The subject information acquisition apparatus according to Claim 2,
   wherein the first moving mechanism (114) moves the supporting member (100) along a linear trajectory on a two-dimensional plane.

7. The subject information acquisition apparatus according to Claim 1,
   wherein the second conversion elements (102) are disposed at positions closer to the position where the subject (103) is placed than the first conversion elements (101).

8. The subject information acquisition apparatus according to Claim 1,
   wherein the plurality of second conversion elements (102) are arrayed on a plane.

9. The subject information acquisition apparatus according to Claim 3,
   wherein the plurality of second conversion elements (102) are arrayed in an outer periphery side region from an opening portion of the bowl portion (100A).

10. The subject information acquisition apparatus according to Claim 9,
    wherein the plurality of second conversion elements (102) is divided into a plurality of groups including at least a first group and a second group, with the first group and the second group being arrayed at different positions in the outer periphery side region.

11. The subject information acquisition apparatus according to Claim 1, further comprising:

    a holding member (701) configured to hold the subject (103);
    wherein the holding member (701) is configured so that the incident angle of the acoustic waves transmitted from the second conversion elements (102) to the holding member (701) is an angle closer to perpendicular to the holding member (701) than a total reflection angle.

12. The subject information acquisition apparatus according to Claim 3,
    wherein the plurality of second conversion elements (102) are arrayed in an arc shape,
    and wherein the plurality of second conversion elements (102) performs transmission of acoustic waves and reception of reflection waves in an inner space of the spherical face.

13. The subject information acquisition apparatus according to Claim 3,
    wherein the plurality of second conversion elements (102) are arrayed in a three-dimensional spiral on the spherical face.

14. The subject information acquisition apparatus according to Claim 2,
wherein the first moving mechanism (114) moves the plurality of first conversion elements (101) and the plurality of second conversion elements (102) together, by moving the supporting member (100).

15. The subject information acquisition apparatus according to Claim 2, further comprising:

a second moving mechanism (104) configured to be capable of moving the plurality of second conversion elements (102) independently from the moving of the supporting member (100).

16. The subject information acquisition apparatus according to Claim 15,
wherein the second moving mechanism (104) moves the plurality of second conversion elements (102) along a linear trajectory on a two-dimensional plane.

17. The subject information acquisition apparatus according to Claim 1, further comprising:

a control unit (118) configured to control each of the light source (110), the plurality of first conversion elements (101), the plurality of second conversion elements (102), and the first moving mechanism (114),
wherein the control unit (118) is capable of executing
a mode in which the first conversion elements (101) are moved while receiving photoacoustic waves at a plurality of positions on the trajectory, and
a mode in which the second conversion elements (102) are moved while transmitting acoustic waves and receiving reflection waves at a plurality of positions on the trajectory,
at mutually different periods.

18. The subject information acquisition apparatus according to Claim 1, further comprising:

a control unit (118) configured to control each of the light source (110), the plurality of first conversion elements (101), the plurality of second conversion elements (102), and the first moving mechanism (114),
wherein the control unit (118) is capable of executing
a mode in which the supporting member (100) is moved while alternately performing each of
reception of photoacoustic waves by the first conversion elements (101) and
transmission of acoustic waves and reception of reflection waves by the second conversion elements (102),
at a plurality of detection positions.

19. The subject information acquisition apparatus according to Claim 18,
wherein the plurality of second conversion elements (102) configure an array (1020), arrayed lined up in a predetermined direction,
and wherein the array (1020) moves as to the supporting member (100) during a moving period of the supporting member (100), so that a longitudinal direction of the array and a movement direction of the supporting member (100) intersect.

20. The subject information acquisition apparatus according to Claim 18,
wherein the plurality of second conversion elements (102) is divided into a plurality of groups,
wherein each of the plurality of groups is an array (1020) where the plurality of second conversion elements (102) are arrayed lined up in a predetermined direction,
and wherein the control unit (118) is capable of selecting second conversion elements (102) in increments of groups to perform transmission of audio waves and reception of reflection waves, in accordance with the movement direction of the supporting member (100).

21. A subject information acquisition apparatus comprising:

a light source (110) configured to generate light;
a plurality of first conversion elements (101), each of which receives photoacoustic waves generated within a subject (103) by the subject (103) being irradiated by the light;
a supporting member (100) configured to support the plurality of first conversion elements (101); and
a plurality of second conversion elements (102) which are different from the first conversion elements (101) and which transmit acoustic waves to the subject (103) and each receive reflection waves reflected within the subject (103);

wherein the plurality of first conversion elements (101) are disposed on the supporting member (100) so that directions, in which reception sensitivity is highest of each first conversion element in at least a part of the plurality of first conversion elements (101), intersect.

# FIG. 1

EP 2 868 279 A1

# FIG. 2

FIG. 3

# FIG. 4

# FIG. 5A

# FIG. 5B

# FIG. 6A

# FIG. 6B

FIG. 7A

FIG. 7B

FIG. 8A

PHOTOACOUSTIC

401     401     401

ULTRASOUND A

402     406

ULTRASOUND B

403     407

FIG. 8B

405

4052

1020B

1020A

1020B

4050

1020A

1020B

1020A

4051

## FIG. 9A

1020

501

## FIG. 9B

100A

1020

501

FIG. 10A

FIG. 10B

FIG. 10C

FIG. 11A

FIG. 11B

FIG. 11C

**Europäisches Patentamt**
**European Patent Office**
**Office européen des brevets**

## EUROPEAN SEARCH REPORT

Application Number

EP 14 18 9130

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2010/030817 A1 (ENDRA INC [US]; THORNTON MICHAEL M [CA]; KRUGER ROBERT A [US]) 18 March 2010 (2010-03-18) | 1-3,5-7, 9-21 | INV. A61B8/00 |
| Y | * abstract; figures 1-4 * <br> * page 3, line 13 - page 4, line 9 * <br> * page 5, line 2 - line 3 * <br> * page 8, line 1 - line 3 * <br> * page 11, line 1 - page 12, line 4 * <br> * the whole document * | 4,8 | ADD. A61B5/00 A61B8/08 |
| X | US 2011/208057 A1 (OIKAWA KATSUYA [JP]) 25 August 2011 (2011-08-25) | 1,8 | |
| Y | * paragraph [0024] - paragraph [0027] * | 8 | |
| A | * paragraph [0031] * <br> * abstract * | 2-7,9-21 | |
| X | US 2012/259198 A1 (NAGAE KENICHI [JP] ET AL) 11 October 2012 (2012-10-11) | 1,8 | |
| A | * abstract * <br> * paragraph [0023] - paragraph [0024] * | 2-7,9-21 | |
| Y | KRUGER ROBERT A ET AL: "Dedicated 3D photoacoustic breast imaging", MEDICAL PHYSICS, AIP, MELVILLE, NY, US, vol. 40, no. 11, 10 October 2013 (2013-10-10), XP012178397, ISSN: 0094-2405, DOI: 10.1118/1.4824317 [retrieved on 2015-02-24] | 4 | |
| A | * pages 113301-7, column 2, line 3 - line 7; figure 5 * <br> * the whole document * | 1-3,5-21 | |

**TECHNICAL FIELDS SEARCHED (IPC)**

A61B

The present search report has been drawn up for all claims

| Place of search | Date of completion of the search | Examiner |
|---|---|---|
| The Hague | 2 March 2015 | Furlan, Stéphane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 14 18 9130

This annex lists the patent family members relating to the patent documents cited in the above-mentioned European search report.
The members are as contained in the European Patent Office EDP file on
The European Patent Office is in no way liable for these particulars which are merely given for the purpose of information.

02-03-2015

| Patent document cited in search report | | Publication date | Patent family member(s) | | Publication date |
|---|---|---|---|---|---|
| WO 2010030817 | A1 | 18-03-2010 | CA | 2736868 A1 | 18-03-2010 |
| | | | CN | 102223840 A | 19-10-2011 |
| | | | EP | 2352422 A1 | 10-08-2011 |
| | | | US | 2011306865 A1 | 15-12-2011 |
| | | | WO | 2010030817 A1 | 18-03-2010 |
| US 2011208057 | A1 | 25-08-2011 | JP | 5448918 B2 | 19-03-2014 |
| | | | JP | 2011172730 A | 08-09-2011 |
| | | | US | 2011208057 A1 | 25-08-2011 |
| US 2012259198 | A1 | 11-10-2012 | CN | 102762153 A | 31-10-2012 |
| | | | EP | 2536338 A1 | 26-12-2012 |
| | | | JP | 2011167228 A | 01-09-2011 |
| | | | US | 2012259198 A1 | 11-10-2012 |
| | | | WO | 2011102105 A1 | 25-08-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010030817 A

- US 5713356 A

**Non-patent literature cited in the description**

- **M. SASSO et al.** Medical Ultrasound Imaging Using The Fully Adaptive Beamformer. *Proc. Acoustics, Speech Signal Process,* March 2005, vol. 2, 489-492

- **HIROFUMI TAKI ; KOUSUKE TAKI ; TAKUYA SAKAMOTO ; MAKOTO YAMAKAWA ; TSUYOSHI SHIINA ; TORU SATO.** *Conf Proc IEEE Eng Med Biol Soc,* 2010, vol. 1, 5298-5301